Europäisches Patentamt

European Patent Office

Office européen des brevets

(19)

(11) Publication number: **0 128 043**
**B1**

(12) # EUROPEAN PATENT SPECIFICATION

(45) Date of publication of patent specification: **25.10.89**

(51) Int. Cl.[4]: **A 61 L 17/00**

(21) Application number: **84303785.4**

(22) Date of filing: **05.06.84**

(54) Treated collagen based surgical threads.

(30) Priority: **06.06.83 US 501040**

(43) Date of publication of application:
**12.12.84 Bulletin 84/50**

(45) Publication of the grant of the patent:
**25.10.89 Bulletin 89/43**

(84) Designated Contracting States:
**AT BE CH DE FR IT LI LU NL SE**

(56) References cited:
**FR-A-2 297 051**
**US-A-3 896 814**
**US-A-4 047 533**

(73) Proprietor: **ETHICON INC.**
**U.S. Route 22**
**Somerville New Jersey 08876 (US)**

(72) Inventor: **Hunter, Alastair Wilson**
**516 Spring Valley Drive**
**Bridgewater, NJ 08807 (US)**
Inventor: **Bogdansky Simon**
**5G Foxwood Drive**
**Morris Plains, NJ 07950 (US)**

(74) Representative: **Jones, Alan John et al**
**CARPMAELS & RANSFORD 43 Bloomsbury Square**
**London, WC1A 2RA (GB)**

Courier Press, Leamington Spa, England.

## Description

The invention relates to collagen based surgical threads containing a nonionic surfactant which enhances the pliability and, in many cases, the tie down performance of the thread.

### Background of the invention

Collagen based surgical threads, in the form of "catgut" sutures and ligatures, have been employed as absorbable surgical threads since Lister's time in the late 19th Century. This invention relates to an improvement in the art of collagen-based surgical threads, whether in the form of surgical gut sutures and ligatures ("gut" sutures and ligatures are actually derived from the intestines of animals such as beef and sheep), or in the form of regenerated collagen sutures and ligatures which may be derived from the tendons of cattle.

Historically, collagen based surgical threads have been supplied to the user in a sterile tube or packet immersed in alcohol containing a small percentage of water. The reason for this is that the liquid was needed in order to impart sufficient flexibility to the thread for it to be readily used by the surgeon.

More recently, it has been proposed to package collagen based sutures containing absorbed liquid that acts as a plasticizer, so that it was not necessary to package the suture immersed in a liquid. For instance, one approach to this is described in U.S.—A—3,896,814. In this approach, the collagen based suture is treated with a liquid containing water, a hygroscopic agent such as glycerol, and then a coating of a hydrophobic material is placed upon the suture. The hydrophobic material is described as being either a silicone or a lipoid.

This invention provides a different approach to providing a collagen-based suture and ligature that need not be immersed in liquid as it is supplied to the surgeon. The sutures and ligatures provided by the invention are actually more pliable than the standard surgical gut now employed commercially, and also, in many cases, exhibit enhanced lubricity and tie-down properties.

### Brief summary of the invention

The invention provides a collagen based surgical thread containing absorbed therein water, a hygroscopic agent and, as a nonionic surfactant, a polyoxyethylene/polyoxypropylene block copolymer in an amount sufficient to enhance the pliability and, in many cases, the tie down performance of the thread. The invention also provides a package containing such a suture, said package being free of extraneous liquid, as well as a process for producing the collagen based surgical thread which comprises contacting a collagen based surgical thread with a solution of water, a hygroscopic agent and, as a nonionic surfactant, a polyoxyethylene/polyoxypropylene block copolymer.

### The prior art

U.S.—A—3,896,814, describes collagen based sutures containing water, a hygroscopic agent, and a coating of a hydrophobic lubricating agent selected from the group consisting of lipoids and silicones.

U.S.—A—4,043,344 and US—A—4,047,533, disclose the use of coatings of a bioabsorbable polyoxyethylene/polyoxypropylene copolymer lubricant on the surface of either a synthetic absorbable suture or a non-absorbable suture.

### Detailed description of the invention

The invention is employed with collagen based sutures and ligatures, either gut sutures and ligatures or regenerated collagen sutures and ligatures. Surgical gut is made from the intestines of animals. For instance, the submucosa layer of sheep intestine and the serosa layer of beef intestines are particularly preferred for producing surgical gut. Also, regenerated collagen sutures and ligatures are made from collagen that has been produced from the tendons of beef cattle. The nature and production of such surgical threads are well known in the art, and need not be repeated here. This invention is employed as one of the final steps prior to sterilization and packaging of such collagen based surgical threads.

The treating solution, called "tubing fluid", that is employed in the invention contains water, a hygroscopic agent that is used for the purpose of retaining the water in the surgical thread, and a nonionic surfactant. Preferably it also contains a lower alkanol such as isopropyl alcohol. The hygroscopic agents contemplated are materials that have a high affinity for water and a low level of volatility, and which are biologically acceptable materials. Such materials include trihydric alkanols, glycols, and low molecular weight polyoxyethylene glycols. Glycerol is the preferred hygroscopic agent, although other materiaels such as diethylene glycol, propylene glycol, and polyoxyethylene glycols having molecular weights up to about 300 or 400 can be employed as the hygroscopic agent. In the aqueous solution of hygroscopic agent, the hygroscopic agent is usually employed in a proportion of from about 20 to 30 weight per cent, based upon total weight of the treating solutiņ. Isopropyl alcohol is usually employed in the treating solution in amounts of from about 25 to 45 weight per cent.

The next ingredient that is employed in the treating solution is a nonionic surfactant. The nonionic surfactants for use in the invention are polyoxyethylene/polyoxypropylene block copolymer surfactants. The nonionic surfactant is employed in the treating solution in a proportion of from about 1 to about 15 weight per cent, and preferably from about 5 to about 12 weight per cent, based upon the total weight of the treating solution.

The treating solution can also contain other materials such as sodium benzoate and N,N-diethylaminoethanol, which are used as corrosion inhibitors.

The collagen based surgical threads that are treated in accordance with the process of the invention are immersed in the above described fluid for a period of time sufficient for the threads to absorb fluid. Normally this will be from about 5 to about 60 minutes, and preferably from about 20 to 40 minutes. The treatment can be carried out at room temperature, i.e., from about 20° to 30°C.

After the surgical thread has been immersed in the treating fluid long enough for it to absorb the fluid, the threads are then removed, drained, and then sealed in a foil package or other similar package and sterilized, preferably by exposure to gamma radiation.

The actual amount of the treating solution that is absorbed by the thread is very difficult to determine with accuracy. However, the desired amount will be absorbed if the foregoing treating instructions are followed.

The following examples illustrates the invention:

Example 1

A treating solution is prepared by mixing the following ingredients:

TABLE I

|  | Parts by weight |
| --- | --- |
| Water | 34 |
| Glycerol | 24 |
| Isopropyl alcohol | 31 |
| "Pluronic" F-68[1] | 10 |
| Sodium benzoate | 0.5 |
| N,N-Diethylaminoethanol | 0.5 |

[1] A polyoxyethylene/polyoxypropylene block copolymer surfactant. It has a molecular weight of about 8350, and is an ABA-type block copolymer. The two A blocks each contain about 75 oxyethylene units; the B block contains about 30 oxypropylene units.

Surgical gut threads are immersed in this fluid for about 30 minutes at room temperature. They are then removed, drained, sealed in a foil package, and sterilized by exposure to $Co^{60}$ irradiation (total dose-about 2.5 mrads).

The surgical gut threads so treated are much more pliable than the present commercial surgical gut threads that are produced in the standard way and packaged in water/isopropyl alcohol solution, as is indicated by a significantly reduced Young's Modulus (75,000±25,000 psi (520±170 MPa) versus 300,000±10,000 psi (2070±70 MPa).

Example 2 and Controls 1 and 2

Paper folders containing Size #1 chromic surgical gut sutures are soaked in the fluids indicated in Table II, below, for 30 minutes at room temperature, are then drained to remove excess fluid, packaged in foil packages, and are sterilized by $Co^{60}$.

TABLE II

| Component | Example 2 | Control 1 |
| --- | --- | --- |
| Isopropyl alcohol | 5.1 liters | 5.1 liters |
| Distilled water | 3.75 liters | 3.75 liters |
| Glycerol | 2.0 liters | 2.0 liters |
| Sodium benzoate | 5.0 grams | 5.0 grams |
| Silicon emulsion[2](1%) | — | 100 ml |
| Diethylaminoethanol | 5.0 ml | 5.0 ml |
| "Pluronic" F-68 (2%) | 200 grams | — |

[2] Dow Corning® 365 Emulsion.

These sutures, along with standard production Size #1 chromic gut sutures packaged in water/isopropyl alcohol (Control 2), are subjected to the tie down-roughness test described in U.S.—A—3,942,532, the disclosure of which is incorporated herein by reference. The results are displayed in Table III:

3

TABLE III

| | Example 2 | Control 1 | Control 2 |
|---|---|---|---|
| Tiedown-roughness, grams | 165 | 440 | 454 |

(Each value given in the table is the average of three tests).

As the foregoing data illustrate, the polyoxyethylene-polyoxypropylene block copolymer surfactant yields a significantly smoother suture that is easier to tie down than either of the two controls.

Example 3 The procedure of Example 2 is followed, except that the proportion of the "Pluronic" F-68 in the tubing fluid is varied from 2 weight per cent to 10 weight per cent. The tie down-roughness test performance of the gut sutures so treated is shown in Table IV:

TABLE IV

| | 2% F-68 | 4% F-68 | 10% F-68 |
|---|---|---|---|
| Tie down-roughness, grams | 220 | 206 | 192 |

**Claims**

1. A collagen-based surgical thread containing absorbed therein water, a hygroscopic agent and, as a nonionic surfactant, a polyoxyethylene/polyoxypropylene block copolymer in an amount sufficient to enhance the pliability of said thread.

2. The collagen-based thread of claim 1 wherein said thread is a surgical gut suture or ligature.

3. The collagen-based surgical thread of claim 1 or claim 2 wherein said hygroscopic agent is glycerol.

4. The collagen-based surgical thread of any one of claims 1 to 3 wherein the thread also contains isopropyl alcohol.

5. A process for producing a flexible collagen based surgical thread having good pliability properties, which comprises immersing a collagen based thread in a solution of water, a hygroscopic agent and, as a nonionic surfactant, a polyoxyethylene/polyoxypropylene block copolymer.

6. The process of claim 5 wherein said solution also contains isopropyl alcohol.

7. A sealed, sterile package containing, in sterile form, the collagen based surgical thread of any one of claims 1 to 4.

**Patentansprüche**

1. Fadenmaterial auf Kollagenbasis für chirurgische Zwecke, welches darin absorbiertes Wasser, ein hygroskopisches Mittel und, als nichtionisches grenzflächenaktives Mittel, ein Polyoxyethylen/Polyoxypropylen-Blockcopolymer in einer Menge enthält, welche ausreicht, um die Geschmeidigkeit des Fadenmaterials zu erhöhen.

2. Fadenmaterial auf Kollagenbasis nach Anspruch 1, wobei das Fadenmaterial ein Darmfadenmaterial oder ein Darmabbindematerial für chirurgische Zwecke ist.

3. Fadenmaterial auf Kollagenbasis für chirurgische Zwecke, nach Anspruch 1 oder 2, worin das hygroskopische Mittel Glycerin ist.

4. Fadenmaterial auf Kollagenbasis für chirurgische Zwecke nach einem der Ansprüche 1 bis 3, wobei das Fadenmaterial auch Isopropylalkohol enthält.

5. Verfahren zur Herstellung eines biegsamen, gute Geschmeidigkeitseigenschaften aufweisenden Fadenmaterials auf Kollagenbasis für chirurgische Zwecke, welches Verfahren das Eintauchen eines Fadenmaterials auf Kollagenbasis in eine Lösung von Wasser, einem hygroskopischen Mittel und, als nichtionischem grenzflächenaktivem Mittel, einem Polyoxyethylen/Polyoxypropylen-Blockcopolymer umfaßt.

6. Verfahren nach Anspruch 5, wobei die Lösung auch Isopropylalkohol enthält.

7. Dicht verschlossene, sterile Packung, welche in steriler Form das Fadenmaterial auf Kollagenbasis für chirurgische Zwecke nach einem der Ansprüche 1 bis 4 enthält.

**Revendications**

1. Fil chirurgical à base de collagène qui contient en absorption, de l'eau, un agent hygroscopique et, en qualité de surfactif non ionique, un copolymère séquencé polyoxyéthylène/polyoxypropylène en une quantité suffisante pour rehausser la souplesse dudit fil.

2. Fil à base de collagène selon la revendication 1, dans lequel ledit fil est une suture ou ligature chirurgicale en boyaux.

3. Fil chirurgical à base de collagène selon la revendication 1 ou 2, dans lequel ledit agent hygroscopique est le glycérol.

4. Fil chirurgical à base de collagène selon l'une quelconque des revendications 1 à 3, dans lequel le fil contient également de l'alcool isopropylique.

5. Procédé de production d'un fil chirurgical flexible à base de collagène possédant de bonnes propriétés de souplesse, qui consiste à immerger un fil à base de collagène dans une solution d'eau, d'un agent hygroscopique et, à titre de surfactif non ionique d'un copolymère séquencé polyoxyéthylène/polyoxypropylène.

6. Procédé selon la revendication 5, dans lequel ladite solution contient également de l'alcool isopropylique.

7. Emballage stérile hermétiquement fermé contenant, sous une forme stérile, un fil chirurgical à base de collagène selon l'une quelconque des revendications 1 à 4.